(19) Europäisches Patentamt
European Patent Office
Office européen des brevets

(11) **EP 3 273 237 B1**

(12) **EUROPÄISCHE PATENTSCHRIFT**

(45) Veröffentlichungstag und Bekanntmachung des Hinweises auf die Patenterteilung:
**29.11.2023 Patentblatt 2023/48**

(21) Anmeldenummer: **17000682.9**

(22) Anmeldetag: **20.05.2014**

(51) Internationale Patentklassifikation (IPC):
**G01N 33/22** (2006.01) **G01N 7/00** (2006.01)

(52) Gemeinsame Patentklassifikation (CPC):
**G01N 7/00; G01N 33/225;** G01N 25/005;
G01N 25/18

(54) **VERFAHREN UND MESSVORRICHTUNG ZUR BESTIMMUNG VON PHYSIKALISCHEN GASEIGENSCHAFTEN**

METHOD AND MEASURING DEVICE FOR DETERMINING PHYSICAL GAS PROPERTIES

PROCÉDÉ ET DISPOSITIF DE MESURE POUR LA DÉTERMINATION DE PROPRIÉTÉS PHYSIQUES DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2013 EP 13002708**

(43) Veröffentlichungstag der Anmeldung:
**24.01.2018 Patentblatt 2018/04**

(62) Dokumentnummer(n) der früheren Anmeldung(en) nach Art. 76 EPÜ:
**14001767.4 / 2 806 271**

(73) Patentinhaber: **MEMS AG**
**5200 BRUGG (CH)**

(72) Erfinder:
• **Prêtre, Philippe**
 **5405 Dättwil (CH)**
• **Kempe, Andreas**
 **8049 Zürich (CH)**
• **Suter, Tobias**
 **8802 Kilchberg (CH)**

(74) Vertreter: **IPS Irsch AG**
 **Langfeldstrasse 88**
 **8500 Frauenfeld (CH)**

(56) Entgegenhaltungen:
EP-A1- 1 265 068   EP-A1- 2 015 056
EP-A2- 0 591 639   WO-A1-99/02964
US-A- 4 527 418

EP 3 273 237 B1

**Beschreibung**

Die Erfindung bezieht sich auf ein Verfahren und auf eine Messvorrichtung zur Bestimmung physikalischer Eigenschaften und brenntechnisch relevanter Grössen von Gasen und Gasgemischen. Unter physikalischen Gaseigenschaften werden insbesondere die Dichte, Wärmeleitfähigkeit, Wärmekapazität und Viskosität und die daraus korrelierbaren, brenntechnisch relevanten Grössen wie der Energieinhalt, Brennwert, Wobbe-Index, die Methanzahl und/oder der Luftbedarf des Gases oder Gasgemisches verstanden.

Bei Gasfeuerungsregelungen ist es wichtig, bei wechselnder Brenngasqualität die Brennerbelastung konstant zu halten. Der entsprechende Index zur Anzeige der Austauschbarkeit von Gasen ist der Wobbe-Index, gebildet aus dem Brennwert und der Wurzel aus dem Dichteverhältnis zwischen Luft und diesem Gas. Bei gleichem Wobbe-Index ergibt sich dann die gleiche Wärmebelastung eines Brenners.

Bei der Regelung von (Erd-)Gasmotoren ist zur Leistungs- bzw. Effizienzsteigerung die Kenntnis des Brennwerts bei wechselnden (Erd-)Gasqualitäten wichtig, zur Beurteilung des Zündverhaltens (Klopfen bzw. Zündaussetzer) wird hingegen bei Gas die zur Oktanzahl bei Benzin analoge Methanzahl herangezogen.

Für einen optimalen Verbrennungsprozess ist das richtige Mischverhältnis zwischen Brenngas und Luft wichtig, der sogenannte Luftbedarf. Bei zuwenig Luft bildet sich normalerweise Russ (Abgase), was vor allem bei Brennstoffzellen zu deren Zerstörung führen kann. Zuviel Luft bei der Verbrennung resultiert in einer kleineren Leistungsausbeute. Dabei hängt der optimale Wert von der jeweiligen Anwendung ab, ändert sich aber wieder bei wechselnder Gasqualität.

Aus der Literatur sind Korrelationsmethoden zur Berechnung brenntechnisch relevanter Größen bekannt, siehe zum Beispiel U. Wernekinck, "Gasmessung und Gasabrechung", Vulkan Verlag, 2009, ISBN 978-3-8027-5620-7. Dabei werden folgende Kombinationen von Messgrössen verwendet:

   A. Dielektrizitätskonstante, Schallgeschwindigkeit, $CO_2$-Gehalt

   B. Schallgeschwindigkeit bei 2 Drücken, $CO_2$-Gehalt

   C. Wärmeleitfähigkeit bei 2 Temperaturen, Schallgeschwindigkeit

   D. Wärmeleitfähigkeit, Wärmekapazität, dynamische Viskosität

   E. Wärmeleitfähigkeit, Infrarot Absorption (nicht dispersiv)

   F. Infrarot Absorption (dispersiv)

Kommerziell erhältliche Geräte, die für eichpflichtige Brennwertmessungen zugelassen sind, gibt es zurzeit nur wenige, z.B. das Gerät EMC500 von RMG-Honeywell (Typus D plus $CO_2$-Gehalt), oder das Gerät Gas-lab Q1 von Elster-Instromet (Typus E plus $CO_2$-Gehalt). Wegen des hohen Einstandspreises ist jedoch keines dieser Geräte für den Massenmarkt geeignet.

Integrierte CMOS-Hitzdrahtanemometer ermöglichen sowohl eine mikrothermische Wärmeleitfähigkeitsmessung als auch eine Massenflussmessung. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen. Sie unterscheidet sich insbesondere von herkömmlichen, thermischen Massenflussmessern durch eine Messung direkt im Gasstrom und nicht von aussen an einer den Gasstrom umfassenden Metallkapillare.

In WO 99/02964 A1 wird ein Verfahren zur Bestimmung der Dichte eines Gases beschrieben, in welchem das Gas aus einem Behälter durch einen kritische Düse fliesst und die Zeit $\Delta t$ für einen bestimmten Druckabfall gemessen wird.

In EP 0 591 639 A2 wird ein Verfahren und eine Vorrichtung zur Bestimmung der Gasdichte beschrieben, wobei zuerst die Gasdichte bei vorgegebenen Bedingungen gemessen wird, um dann mittels eines Volumenkorrekturverhältnisses die Gasdichte bei Flussbedingungen zu ermitteln.

Aus EP 2 015 056 A1 ist ein thermischer Durchflusssensor zur Bestimmung einer brenntechnisch relevanten Grösse bekannt, basierend auf einer Wärmeleitfähigkeitsmessung bei quasi bekanntem Massenfluss. Dazu wird eine kritische Düse benutzt, um den Massenfluss konstant zu halten, und es wird versucht, die Gasartenabhängigkeit der kritischen Düse mittels der Wärmeleitfähigkeit zu korrigieren. Die Information zur Korrelation brenntechnisch relevanter Grössen ist jedoch auf zwei quasi unabhängige Messwerte beschränkt, sodass keine Validierung der Messdaten möglich ist.

Aus WO 2004/036209 A1 ist ein Sensor zur Bestimmung brenntechnisch relevanter Grössen bekannt, bei dem der Massenfluss konstant gehalten wird und mittels einer thermischen Messung eine zur Wärmekapazität proportionale Grösse ermittelt wird. Da es sich nicht um einen mikrothermischen Sensor handelt, kann nicht auf die Wärmeleitfähigkeit geschlossen werden, womit die Bestimmung der Wärmekapazität und der daraus abgeleiteten, brenntechnisch relevanten Grössen nur bis auf einen Proportionalitätsfaktor möglich ist, was eine zusätzliche Kalibrierung mit bekannten Gaszusammensetzungen notwendig macht. Zudem entfällt die Information über die Wärmeleitfähigkeit und damit die Möglichkeit der Korrelation der Wärmeleitfähigkeit $\lambda$ mit einer der brenntechnisch relevanten Grössen. Im Weiteren ist die Genauigkeit dieser Methode limitiert durch die auftretenden Änderungen der nicht zugänglichen Wärmeleitfähigkeit $\lambda$.

Der Erfindung liegt daher die Aufgabe zu Grunde, ein Verfahren und eine Messvorrichtung zur Bestimmung physikalischer Eigenschaften von Gasen und Gasgemischen anzugeben, mit denen eine höhere Genauigkeit erzielt werden kann als mit den Sensoren aus den oben beschriebenen Patentdokumenten, wobei es möglich ist, die Messvorrichtung zu tieferen Kosten herzustellen als kommerziell erhältliche Geräte, die für eichpflichtige Brennwertmessungen zugelassen sind.

Die Aufgabe wird durch ein Verfahren nach Anspruch 1 und durch eine Messvorrichtung nach Anspruch 6 gelöst.

Druckabfallmessung eines vorgegebenen Volumens an Gas durch eine kritische Düse:

Der Massenfluss $\dot{m}$ durch eine kritische Düse ist gegeben durch

$$\dot{m} = C_d \cdot p \cdot A^* \cdot \psi_{max} \cdot \sqrt{\frac{M}{T \cdot R_m}} \,, \qquad (1)$$

worin $C_d$ den "Discharge Coefficient", d.h. den Verlustfaktor einer realen gegenüber einer idealen kritischen Düse, $p$ den Vordruck, $A^*$ den Düsenquerschnitt, $T$ die Vortemperatur, $R_m$ die universelle Gaskonstante, $M$ das Molekulargewicht des Gases und $\psi_{max}$ den Maximalwert der Ausflussfunktion bezeichnet. Letztere ist eine Funktion des isentropen Koeffizienten $\gamma = c_p/c_V$ (Verhältnis von isobarer zu isochorer Wärmekapazität),

$$\psi = \sqrt{\gamma} \cdot \left(\frac{\gamma+1}{2}\right)^{\frac{\gamma+1}{2(1-\gamma)}}. \qquad (2)$$

Lässt man aus einem bekanntem Volumen $V$ das Gas von einem hohen Druck über die kritische Düse entspannen (z.B. von 9 auf 4 bar), ist der Druck in dem Volumen aufgrund des idealen Gasgesetzes wie folgt von der Zeit $t$ abhängig:

$$p(t) = m(t) \cdot \frac{R_m \cdot T}{M \cdot V}. \qquad (3)$$

Die Änderungsrate des Druckes ergibt sich somit zu

$$\frac{dp(t)}{dt} = \frac{dm(t)}{dt} \cdot \frac{R_m \cdot T}{M \cdot V} = \dot{m}(t) \cdot \frac{R_m \cdot T}{M \cdot V} \qquad (4)$$

und zusammen mit Gleichung (1) zu

$$\frac{dp(t)}{dt} = C_d \cdot p \cdot A^* \cdot \psi_{max} \cdot \sqrt{\frac{M}{T \cdot R_m}} \cdot \frac{R_m \cdot T}{M \cdot V}$$

$$= C_d \cdot \frac{A^* \cdot \psi_{max}}{V} \cdot \sqrt{\frac{R_m \cdot T}{M}} \cdot p(t). \qquad (5)$$

Misst man also den Verlauf des Druckes in Abhängigkeit der Zeit, kann man so die Zeitkonstante $\tau$ der durch

Integration erhaltenen, zugehörigen Exponentialfunktion bestimmen:

$$1/\tau \;=\; \frac{C_d \cdot A^* \cdot \psi_{\max}}{V} \cdot \sqrt{\frac{R_m \cdot T}{M}} \cdot \qquad (6)$$

Kennt man durch Messen zusätzlich die Temperatur $T$, kann man durch Weglassen aller nicht gasabhängigen Grössen einen Gaseigenschaftsfaktor

$$\Gamma^* \;:=\; C_d \cdot \psi_{\max} \cdot \sqrt{\frac{1}{M}} \qquad (7)$$

definieren.

Lässt man umgekehrt das Gas von einem höheren Druck über die kritische Düse in ein bekanntes Volumen $V$ entspannen (z.B. von Umgebungsdruck nach Vakuum), lautet Gleichung (5') für die Druckzunahme im Volumen $V$ wie folgt:

$$\frac{dp(t)}{dt} = C_d \cdot p_{Nozzle} \cdot A^* \cdot \psi_{\max} \cdot \sqrt{\frac{M}{T \cdot R_m}} \cdot \frac{R_m \cdot T}{M \cdot V}$$
$$= C_d \cdot \frac{A^* \cdot \psi_{\max}}{V} \cdot \sqrt{\frac{R_m \cdot T}{M}} \cdot p_{Nozzle} \qquad , \quad (5')$$

wobei in diesem Fall der Druck vor der Düse, $p_{Nozzle}$, konstant ist, was zu einem mit der Zeit linearen Druckanstieg im Volumen $V$ führt mit

$$\frac{C_d \cdot A^* \cdot \psi_{\max}}{V} \cdot \sqrt{\frac{R_m \cdot T}{M}} \cdot p_{Nozzle} \qquad (6')$$

als Proportionalitätskonstante. Kennt man durch Messen zusätzlich die Temperatur $T$ und den Düsenvordruck $p_{Nozzle}$, kann man durch Weglassen aller nicht gasabhängigen Grössen wiederum den Gaseigenschaftsfaktor

$$\Gamma^* \;:=\; C_d \cdot \psi_{\max} \cdot \sqrt{\frac{1}{M}} \qquad (7')$$

definieren.

Massenflussmessung mittels mikrothermischen Sensors:

Zur Beschreibung der mikrothermischen Massenflussmessung wird von der das mikrothermische System beschreibenden, eindimensionalen Wärmeleitungsgleichung ausgegangen (Kerson Huang: Statistical Mechanics, 2. Auflage, John Wiley & Sons, New York 1987, ISBN 0-471-85913-3):

**4**

$$\frac{c_p}{\lambda} \cdot \rho\, v_x \cdot \frac{d}{dx}T = \nabla^2 T + \frac{1}{\lambda}\Theta \ , \qquad (8)$$

wobei

$v_x$ die Komponente der mittleren Fliessgeschwindigkeit (Geschwindigkeitsvektor) $\vec{v}$ in x-Richtung, d. h. entlang des Gasstromes,

$T$ die Temperatur,

$$\frac{d}{dx}T$$

der Temperaturgradient,

$c_p$ die Wärmekapazität des Gases bei konstantem Druck,

$\rho$ die Dichte,

$\lambda$ die Wärmeleitfähigkeit des Gases,

$\nabla^2 T$ der Laplace-Operator, angewandt auf die Temperatur $T$, wobei

$$\nabla^2 = \left(\frac{d}{d_x}\right)^2 + \left(\frac{d}{dy}\right)^2 + \left(\frac{d}{dz}\right)^2 \ ,$$

bedeuten. Da das Gas (Gasstrom) nur in x-Richtung fliesst, werden die Komponenten $v_y$ bzw. $v_z$ in y-Richtung bzw. in z-Richtung der mittleren Fliessgeschwindigkeit $\vec{v}$ zu Null angenommen. $\Theta$ mit der Einheit Watt/m³ beschreibt den Quellterm des Heizelements. Der Quellterm rührt bei der mikrothermischen Methode vom Heizdraht eines miniaturisierten, integrierten Hitzdrahtanemometers her, der Wärmeenergie in das System speist. Die Lösung der Gleichung (8), die die Temperaturverteilung im mikrothermischen System beschreibt, erlaubt es, durch das Messen dieser Temperaturverteilung den Faktor S,

$$S := \frac{c_p}{\lambda} \cdot \rho \cdot v_x \ = \ \frac{c_p}{\lambda} \cdot \frac{\dot{m}}{A} \ , \qquad (9)$$

zu bestimmen, wobei $A$ den Querschnitt des Flusskanals über dem mikrothermischen Sensor bezeichnet. In Kombination mit der kritischen Düse, d.h. durch Anordnen des mikrothermischen Sensors nach der kritischen Düse, ist der Massenfluss aber durch Gleichung (1) gegeben, weswegen

$$\frac{c_p}{\lambda} \cdot \rho \cdot v_x \ = \ \frac{c_p}{\lambda} \cdot C_d \cdot p \cdot \frac{A^*}{A} \cdot \psi_{max} \cdot \sqrt{\frac{M}{T \cdot R_m}} \ . \qquad (10)$$

Mit dem Messen von Druck $p$ und Temperatur $T$ und wiederum durch Weglassen aller gasunabhängigen Grössen erhält man einen zweiten Gaseigenschaftsfaktor

$$\Gamma \ = \ \frac{c_p}{\lambda} \cdot C_d \cdot \psi_{max} \cdot \sqrt{M} \ . \qquad (11)$$

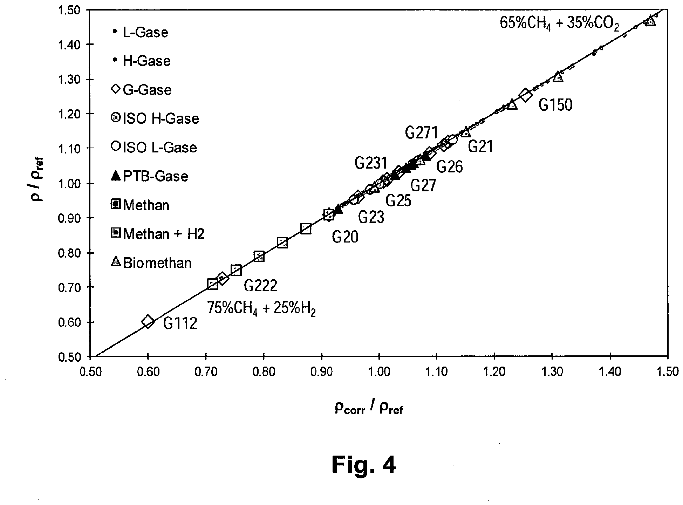

Das Weglassen aller gasunabhängigen Grössen in Gleichung (7) und (11) geschieht implizit, wenn man $\Gamma$ und $\Gamma^*$ ins Verhältnis zu $\Gamma$ und $\Gamma^*$ eines bekannten (Kalibrier-)Gases setzt. Siehe auch Fig. 4.

Messung der Wärmeleitfähigkeit mittels mikrothermischen Sensors:

Man beachte, dass die Wärmeleitfähigkeit $\lambda$ wegen des Quellterms $\Theta$ zusätzlich separat auf die Lösung der Gleichung (8) einwirkt. Umgekehrt kann die Wärmeleitfähigkeit bestimmt werden, wenn der mikrothermische Sensor

ohne beaufschlagten Massenfluss ($v_x$ =0 bzw. $\dot{m}$ = 0) gemessen wird. Die zugehörige Differentialgleichung für die Temperaturverteilung lautet dann einfach

$$\nabla^2 T = -\frac{1}{\lambda}\Theta \ . \tag{12}$$

Validierung der Gaseigenschaftsfaktoren $\Gamma$ bzw. $\Gamma^*$:

Das Verhältnis der beiden Gaseigenschaftsfaktoren $\Gamma$ und $\Gamma^*$ ergibt

$$\Gamma\big/\Gamma^* = \frac{c_p}{\lambda}\cdot M \ \propto \ \frac{c_p}{\lambda}\cdot\rho_{norm} \ , \tag{13}$$

da das Molekulargewicht wegen des für die meisten Gase praktisch identischen Molvolumens proportional zur Normdichte (Dichte bei Normbedingungen 1013.25 mbar und 273.15 K) ist. Damit kann aus dem mittels mikrothermischen Sensors gemessenen Faktor S in Gleichung (9) die Fliessgeschwindigkeit $v_x$ und zusammen mit dem Flusskanalquerschnitt A der Normvolumenfluss $\phi_{norm}$ = $v_x \cdot A$ extrahiert werden. Integration dieses Volumenflusses über die Zeit, d.h. im Zeitintervall $t_2$-$t_1$, sollte dann mit dem aus den entsprechenden Druck- und Temperaturwerten berechneten, abgeflossenen Gasvolumen übereinstimmen:

$$\int_{t_1}^{t_2}\phi_{norm}(t)\,dt \ \overset{!}{=} \ \frac{(p(t_2)-p(t_1))}{1013.25\ mbar}\cdot\frac{273.15\ K}{T}\cdot V \ . \tag{14}$$

Stimmen die zwei Werte nicht überein, kann je nachdem, welche Grösse weniger genau gemessen werden kann, der Normvolumenfluss oder das Drucksignal soweit korrigiert werden, bis Gleichung (14) erfüllt ist. Im Fall einer Normvolumenflusskorrektur für $v_x$ = $\phi_{norm}$ / $A$ erfährt die rechte Seite von Gleichung (13) über den gemessenem Faktor S in Gleichung (9) ebenfalls eine Korrektur und damit auch der Gaseigenschaftsfaktor $\Gamma$ wiederum über Gleichung (13). Im Fall einer Drucksignalkorrektur folgt eine korrigierte Zeitkonstante $\tau$ in Gleichung (6) bzw. eine korrigierte Proportionalitätskonstante in Gleichung (6') und damit eine Korrektur des Gaseigenschaftsfaktor $\Gamma^*$ in Gleichung (7) bzw. (7'). Auf diese Weise sind $\Gamma$ und $\Gamma^*$ konsistent bestimmt worden, denn der Massenfluss durch die Düse ist derselbe wie der Massenfluss, mit dem der mikrothermische Sensor beaufschlagt wird.

Korrelation brenntechnisch relevanter Grössen:

Mit der Messungen der Gaseigenschaftsfaktoren $\Gamma$ und $\Gamma^*$ sowie der Wärmeleitfähigkeit $\lambda$ stehen drei unabhängige Messgrössen zur Verfügung, mit denen nun brenntechnisch relevante Grössen Q mittels einer Funktion $f_{corr}$ korreliert werden können:

$$Q_{corr} = f_{corr}(\Gamma,\Gamma^*,\lambda) \ . \tag{15}$$

Beispielsweise ergibt sich für die Korrelation des in Fig. 4 gezeigten Dichteverhältnisses $\rho_{corr}$ / $\rho_{ref}$ bei 0°C und 1013.25 mbar folgende Korrelationsfunktion:

$$\rho_{corr}\big/\rho_{ref} = f_{corr}(\Gamma,\Gamma^*,\lambda) = \Gamma^r\cdot\Gamma^{*\,s}\cdot\lambda^{\,t} \tag{16}$$

mit Exponenten $r$ = -0.2, $s$ = -1.8 und $t$ = -0.2 und einem typischen H-Erdgas als Referenz.

Verfahren und Messvorrichtung gemäss vorliegender Erfindung

In dem Verfahren zur Bestimmung physikalischer Eigenschaften und/oder brenntechnisch relevanter Grössen eines Gases und Gasgemisches gemäss vorliegender Erfindung:

- fliesst das Gas oder Gasgemisch aus einem Gasreservoir durch eine kritische Düse und über einen mikrothermischen Sensor, wobei die kritische Düse und der mikrothermischen Sensor mit demselben Massenfluss beaufschlagt werden;
- wobei der Druckabfall im Gasreservoir als Funktion der Zeit gemessen wird;
- aus den Messwerten des Druckabfalls ein erster, von einer ersten Gruppe von physikalischen Eigenschaften des Gases oder Gasgemisches abhängiger Gaseigenschaftsfaktor F* bestimmt wird, wobei der erste Gaseigenschaftsfaktor aus einer Zeitkonstante des Druckabfalls abgeleitet und von einem exponentiellen Abfall des gemessenen Druckes ausgegangen wird;
- aus dem Durchflusssignal des mikrothermischen Sensors ein zweiter, von einer zweiten Gruppe von physikalischen Eigenschaften des Gases oder Gasgemisches abhängiger Gaseigenschaftsfaktor $\Gamma$ bestimmt wird, wobei der zweite Gaseigenschaftsfaktor zum Beispiel die Wärmekapazität $c_p$ des Gases oder Gasgemisches enthält oder von derselben abhängig ist;
- mit Hilfe des mikrothermischen Sensors die Wärmeleitfähigkeit $\lambda$ des Gases oder Gasgemisches bestimmt wird; und
- aus dem ersten und/oder zweiten Gaseigenschaftsfaktor F*, $\Gamma$ und der Wärmeleitfähigkeit $\lambda$ mittels Korrelation eine gesuchte physikalische Eigenschaft oder brenntechnisch relevante Grösse ermittelt wird.

Dabei wird von einem exponentiellen Abfall des gemessenen Druckes ausgegangen und der erste Gaseigenschaftsfaktor $\Gamma$* aus der Zeitkonstante des Druckabfalls abgeleitet, wobei der erste Gaseigenschaftsfaktor gebildet wird, indem zum Beispiel zusätzlich die Temperatur T gemessen und alle nicht gasabhängigen Grössen weggelassen werden.

Der Druck im Gasreservoir zu Beginn der Druckabfallmessung ist typisch grösser als der kritische Druck $p_{krit}$ der kritischen Düse und der Aussendruck nach der kritischen Düse kleiner als die Hälfte des kritischen Druckes, oder der Druck im Gasreservoir zu Beginn der Druckzunahmemessung ist typisch kleiner als die Hälfte des kritischen Druckes $p_{krit}$ der kritischen Düse und der Druck vor der kritischen Düse grösser ist als der kritische Druck. Unabhängig von der Ausführungsform und -variante ist das Gasreservoir während der Messung typisch abgetrennt von der Gasversorgung. Das Volumen des Gasreservoirs wird vorteilhafterweise so gewählt, dass der Druck im Gasreservoir bis zum Ende der Messung deutlich ab- oder zunimmt, beispielsweise um mindestens einen Zehntel oder Fünftel des ursprünglichen Druckes.

Weiter umfasst die Erfindung auch die Verwendung eines Gasreservoirs und einer kritischen Düse zur Bestimmung physikalischer Eigenschaften und/oder brenntechnisch relevanter Grössen eines Gases oder Gasgemisches, wobei das Gas oder Gasgemisch unter Druck aus dem Gasreservoir durch die kritische Düse fliesst, wobei der Druckabfall im Gasreservoir als Funktion der Zeit gemessen wird, aus den Messwerten des Druckabfalls ein von physikalischen Eigenschaften des Gases oder Gasgemisches abhängiger Gaseigenschaftsfaktor $\Gamma$* bestimmt wird, der aus einer Zeitkonstante des Druckabfalls abgeleitet wird, und aus dem Gaseigenschaftsfaktor $\Gamma$* mittels Korrelation eine gesuchte physikalische Eigenschaft oder brenntechnisch relevante Grösse ermittelt wird.

In einer anderen vorteilhaften Ausführungsform wird ein Unterdruck im Gasreservoir erzeugt, und das Gas oder Gasgemisch fliesst unter Druck durch die kritische Düse in das Gasreservoir, wobei die Druckzunahme im Gasreservoir als Funktion der Zeit gemessen und aus den Messwerten der Druckzunahme ein von physikalischen Eigenschaften des Gases oder Gasgemisches abhängiger Gaseigenschaftsfaktor F* bestimmt wird, aus dem mittels Korrelation eine gesuchte physikalische Eigenschaft oder brenntechnisch relevante Grösse ermittelt wird.

Die oben beschriebene Verwendung eines Gasreservoirs und einer kritischen Düse zur Bestimmung physikalischer Eigenschaften und/oder brenntechnisch relevanter Grössen eines Gases oder Gasgemisches beziehungsweise das entsprechende Verfahren, in dem ein Gasreservoir und eine kritischen Düse zur Bestimmung physikalischer Eigenschaften und/oder brenntechnisch relevanter Grössen eines Gases oder Gasgemisches verwendet werden, kann auch als eigenständige Erfindung betrachtet werden, die zusätzlich eine Messvorrichtung mit Auswerteeinheit, Gasreservoir und kritischer Düse umfassen kann, wobei die Auswerteeinheit für die Verwendung des Gasreservoirs und der kritischen Düse zur Bestimmung physikalischer Eigenschaften und/oder brenntechnisch relevanter Grössen eines Gases oder Gasgemisches beziehungsweise zur Ausführung des entsprechenden Verfahrens eingerichtet ist.

Weitere Vorteile sind aus der nachstehenden Beschreibung ersichtlich.

Die Erfindung wird im Folgenden anhand der Zeichnungen näher erläutert. Es zeigen:

Fig. 1a    ein Ausführungsbeispiel des schematischen Aufbaus einer Messvorrichtung gemäss eines Beispiels, welches nicht Teil der Erfindung ist, (Hochdruckvariante),

Fig. 1b    eine Ausführungsvariante zu dem in Fig. 1a gezeigten Beispiels, welches nicht Teil der Erfindung ist,

Fig. 2 ein zweites Ausführungsbeispiele des schematischen Aufbaus einer Messvorrichtung gemäss eines Beispiels, welches nicht Teil der Erfindung ist, (Niederdruckvariante),

Fig. 3 ein Ausführungsbeispiel eines mikrothermischen Sensors zur Verwendung in einer Messvorrichtung gemäss eines Beispiels, welches nicht Teil der Erfindung ist, und

Fig. 4 eine grafische Darstellung des direkt gemessenen Dichteverhältnisses (y-Achse) in Abhängigkeit des korrelierten Dichteverhältnisses (x-Achse) für verschieden Gasgruppen bei Normbedingungen (0°C, 1013.25 mbar).

Fig. 5a ein Ausführungsbeispiel des schematischen Aufbaus einer Messvorrichtung gemäss einer zweiten Ausführungsform der Erfindung (Hochdruckvariante),

Fig. 5b eine Ausführungsvariante zu dem in Fig. 5a gezeigten Ausführungsbeispiel,

Fig. 6 ein zweites Ausführungsbeispiele des schematischen Aufbaus einer Messvorrichtung gemäss der zweiten Ausführungsform der Erfindung (Niederdruckvariante),

Fig. 7 eine grafische Darstellung des direkt gemessenen Methananteils (y-Achse) in Abhängigkeit des korrelierten Methananteils (x-Achse) für ein binäres Rohbiogas (Methan und Kohlendioxid).

Fig. 8a ein Ausführungsbeispiel des schematischen Aufbaus einer Messvorrichtung gemäss eines Beispiels, welches nicht Teil der Erfindung ist, mit Gasreservoir und mikrothermischem Sensor (Hochdruckvariante),

Fig. 8b eine Ausführungsvariante zu dem in Fig. 8a gezeigten Ausführungsbeispiel,

Fig. 9 ein zweites Ausführungsbeispiele des schematischen Aufbaus einer Messvorrichtung gemäss eines Beispiels, welches nicht Teil der Erfindung ist, mit Gasreservoir und mikrothermischem Sensor (Niederdruckvariante),

Fig. 10 eine grafische Darstellung der Klasseneinteilung von Erdgasgemischen anhand der Wärmediffusivität (y-Achse) bei gleichzeitiger Kenntnis der Wärmeleitfähigkeit $\lambda$ (x-Achse).

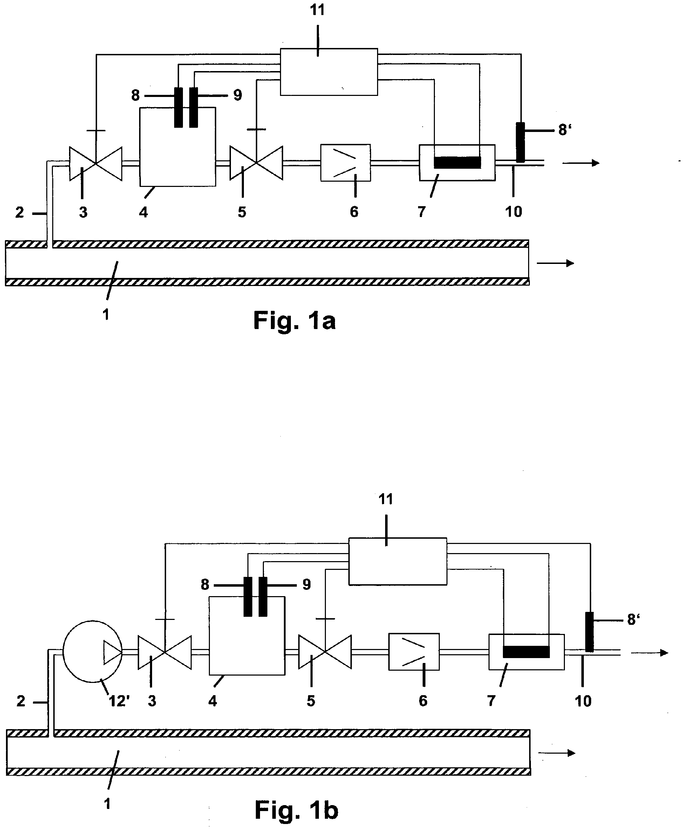

Fig. 1a zeigt ein Ausführungsbeispiel des schematischen Aufbaus einer Messvorrichtung gemäss vorliegender Erfindung im Fall, in dem die Gashauptleitung 1 einen Druck aufweist, der höher als der kritische Druck für die kritische Düse 6 der Messvorrichtung ist (Hochdruckvariante). Im Ausführungsbeispiel umfasst die Messvorrichtung zusätzlich zur kritischen Düse 6 eine Auswerteeinheit 11, die zur Ausführung eines Verfahrens gemäss vorliegender Erfindung eingerichtet ist, ein Gasreservoir 4, das mit einem Drucksensor 8 versehen ist, und einen mikrothermischen Sensor 7 zur Messung des Durchflusses und der Wärmeleitfähigkeit, wobei das Gasreservoir 4 für die Messung mit der kritischen Düse 6 und dem mikrothermischen Sensor 7 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel, welches nicht Teil der Erfindung ist, wird im Folgenden anhand von Fig. 1a beschrieben. In dem Verfahren fliesst das Gas oder Gasgemisch aus dem Gasreservoir 4 durch die kritische Düse 6 und über den mikrothermischen Sensor 7, wobei die kritische Düse und der mikrothermischen Sensor mit demselben Massenfluss beaufschlagt werden. Der Druckabfall im Gasreservoir 4 wird als Funktion der Zeit gemessen und aus den Messwerten des Druckabfalls ein erster, von einer ersten Gruppe von physikalischen Eigenschaften des Gases oder Gasgemisches abhängiger Gaseigenschaftsfaktor $\Gamma^*$ bestimmt, wobei der erste Gaseigenschaftsfaktor zum Beispiel aus einer Zeitkonstante des Druckabfalls abgeleitet wird. Aus dem Durchflusssignal des mikrothermischen Sensors 7 wird ein zweiter, von einer zweiten Gruppe von physikalischen Eigenschaften des Gases oder Gasgemisches abhängiger Gaseigenschaftsfaktor $\Gamma$ bestimmt, wobei der zweite Gaseigenschaftsfaktor zum Beispiel die Wärmekapazität $c_p$ des Gases oder Gasgemisches enthält oder von derselben abhängig ist. Weiter wird mit Hilfe des mikrothermischen Sensors 7 die Wärmeleitfähigkeit $\lambda$ des Gases oder Gasgemisches bestimmt und aus dem ersten und/oder zweiten Gaseigenschaftsfaktor $\Gamma^*$, $\Gamma$ und der Wärmeleitfähigkeit $\lambda$ mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet. Das Einlassventil und Auslassventil können über eine Steuereinheit geöffnet werden. Fallweise kann auch die Auswerteeinheit 11, wie in Fig. 1a gezeigt, die Steuerung des Einlassventils und Auslassventils übernehmen. Dann wird das Auslassventil 5 geschlossen und das Gasreservoir 4, dessen Volumeninhalt V bekannt ist, füllt sich, bis das Einlassventil 3 geschlossen wird. Druck *p* und Temperatur *T* im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} = \frac{p}{1013.25\,mbar} \cdot \frac{273.15\,K}{T} \cdot V \quad . \qquad (17)$$

Ist der Druck *p* im Gasreservoir 4 grösser als der Druck $p_{krit}$, der benötigt wird, damit die Düse 6 kritisch betrieben werden kann, kann das Auslassventil 5 wieder geöffnet werden. Vorzugsweise ist der Druck *p* im Gasreservoir um mehrere bar höher als $p_{krit}$, sodass die Druckabfallmessung über diesen Bereich der Drucküberhöhung vollzogen werden kann, ohne dass die Düse 6 nicht mehr kritisch betrieben ist. Jetzt wird das Auslassventil 5 wieder geschlossen, womit die Druckabfallmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Druckabfallmessung ist der zeitabhängige Druck *p(t)* und die zeitabhängige Temperatur *T(t)* im Druckreservoir 4 gemessen und von der Auswerteeinheit 11 aufgezeichnet worden. Mit diesen Daten wird in der Auswerteeinheit die Zeitkonstante $\tau$ in Gleichung (6) bzw. der Gaseigenschaftsfaktor $\Gamma^*$ in Gleichung (7) bestimmt. Gleichzeitig sind mit dem mikrothermischen Sensor 7 Durchflussdaten gemessen worden, die wiederum die Auswerteeinheit aufgezeichnet hat, um den Faktor S in Gleichung (9) bzw. den Gaseigenschaftsfaktor $\Gamma$ in Gleichung (11) zu bestimmen. Da Ein- und Auslassventil nach der Druckabfallmessung geschlossen sind, fliesst kein Gas mehr über den mikrothermischen Sensor 7. Jetzt kann die Messung der Wärmeleitfähigkeitsmessung $\lambda$ erfolgen. Wiederum von der Auswerteeinheit aufgezeichnet, wird die Wärmeleitfähigkeit $\lambda$ mit Hilfe der Lösung von Gleichung (12) bestimmt.

In der Auswerteeinheit 11 erfolgt nun die (fakultative) Validierung des Gaseigenschaftsfaktors $\Gamma$ bzw. $\Gamma^*$ und danach, je nach gewünschter, brenntechnisch relevanter Grösse Q, deren Berechnung anhand der Gleichung (15) mit zuvor ermittelter Korrelationsfunktion $Q_{corr} = f_{corr}(\Gamma, \Gamma^*, \lambda)$.

Bei Bedarf kann zudem, wie in Fig. 1b gezeigt, ein Verdichter 12' vorgesehen sein, der beispielsweise einlassseitig des Gasreservoirs 4 angeordnet ist, um den Druck im Gasreservoir zu erhöhen.

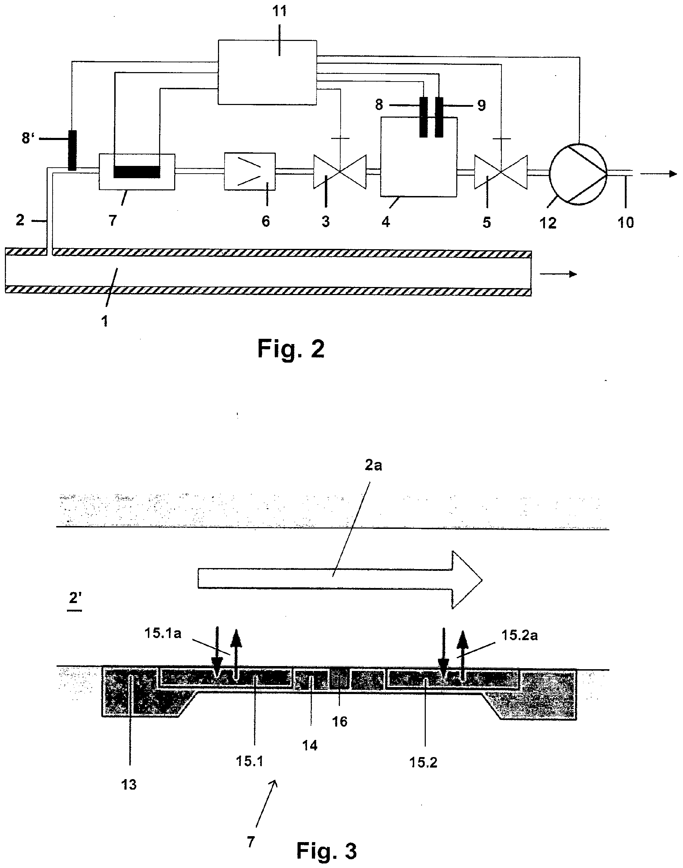

Fig. 2 zeigt ein zweites Ausführungsbeispiel des schematischen Aufbaus einer Messvorrichtung, welche nicht Teil der Erfindung ist, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss vorliegender Erfindung eingerichtet ist, eine kritische Düse 6, und einen mikrothermischen Sensor 7 zur Messung des Durchflusses und der Wärmeleitfähigkeit, wobei das Gasreservoir 4 für die Messung mit der kritischen Düse 6 und dem mikrothermischen Sensor 7 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein Ausführungsbeispiel des Verfahrens zur Bestimmung physikalischer Eigenschaften und/oder brenntechnisch relevanter Grössen eines Gases und Gasgemisches, welches nicht Teil der Erfindung ist, wird im Folgenden anhand von Fig. 2 beschrieben. In dem Verfahren fliesst das Gas oder Gasgemisch unter Druck durch die kritische Düse 6 und über den mikrothermischen Sensor 7 in das Gasreservoir 4, wobei die kritische Düse und der mikrothermischen Sensor mit demselben Massenfluss beaufschlagt werden. Die Druckzunahme im Gasreservoir 4 wird als Funktion der Zeit gemessen und aus den Messwerten der Druckzunahme ein erster, von einer ersten Gruppe von physikalischen Eigenschaften des Gases oder Gasgemisches abhängiger Gaseigenschaftsfaktor $\Gamma^*$ bestimmt, wobei der erste Gaseigenschaftsfaktor zum Beispiel aus einer Proportionalitätskonstante der Druckzunahme abgeleitet wird. Aus dem Durchflusssignal des mikrothermischen Sensors 7 wird ein zweiter, von einer zweiten Gruppe von physikalischen Eigenschaften des Gases oder Gasgemisches abhängiger Gaseigenschaftsfaktor $\Gamma$ bestimmt, wobei der zweite Gaseigenschaftsfaktor zum Beispiel die Wärmekapazität $c_p$ des Gases oder Gasgemisches enthält oder von derselben abhängig ist. Weiter wird mit Hilfe des mikrothermischen Sensors 7 die Wärmeleitfähigkeit $\lambda$ des Gases oder Gasgemisches bestimmt und aus dem ersten und/oder zweiten Gaseigenschaftsfaktor $\Gamma^*$, $\Gamma$ und der Wärmeleitfähigkeit $\lambda$ mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise wird der Druck im Gasreservoir 4 vorgängig soweit erniedrigt, zum Beispiel mit einer Vakuumpumpe 12, dass die kritische Düse 6 kritisch betrieben werden kann, d.h. der Druck im Gasreservoir weniger als die Hälfte des Drucks vor der kritischen Düse beträgt. Es ist kein Hochvakuum erforderlich: Solang der Druck $p$ und die Temperatur $T$ im Gasreservoir 4 gemessen werden, kann immer berechnet werden, welches Normvolumen an Gas in das Gasreservoir geflossen ist. Es ist jedoch von Vorteil, wenn der Druck um einen Faktor niedriger ist als es für kritische Verhältnisse nötig wäre, da dann entsprechend länger gemessen werden kann, was eine genauere Bestimmung der Proportionalitätskonstante ermöglicht.

Für weitere Einzelheiten zum Verfahren, die bei Bedarf verwendet werden können, wird auf die Beschreibung des ersten Ausführungsbeispiels verwiesen, wobei gegebenenfalls der Begriff "Druckabfall" durch "Druckzunahme" ersetzt werden muss.

Fig. 3 zeigt ein Ausführungsbeispiel eines mikrothermischen Sensors zur Verwendung in einer Messvorrichtung, welche nicht Teil der Erfindung ist. Der mikrothermische Sensor 7 kann beispielsweise, wie in Fig. 3 gezeigt, ein integriertes, mikrothermisches CMOS-Hitzdrahtanemometer sein, das im Einsatz in einem Abschnitt 2' der Messleitung angeordnet und mit einem Gas- oder Gasgemischstrom 2a beaufschlag werden kann. Das mikrothermische CMOS-Hitzdrahtanemometer umfasst ein Substrat 13, das typisch eine Membran 14 von wenigen Mikrometer Dicke enthält. Weiter umfasst das CMOS-Hitzdrahtanemometer zwei Thermoelemente 15.1, 15.2 und ein Heizelement 16, das in Flussrichtung zwischen den beiden Thermoelementen angeordnet sein kann. Mit den beiden Thermoelementen 15.1, 15.2 kann die Temperatur erfasst werden, die sich auf Grund des Wärmeaustausches 15.1a, 15.2a mit dem Gas- oder Gasgemischstrom 2a einstellt.

Für weitere Einzelheiten zur Funktionsweise des integrierten, mikrothermischen CMOS-Hitzdrahtanemometers wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 verwiesen.

Fig. 4 zeigt eine Darstellung des direkt gemessenen Dichteverhältnisses $\rho / \rho_{ref}$ (y-Achse) in Abhängigkeit des korrelierten Dichteverhältnisses $\rho_{corr} / \rho_{ref}$ (x-Achse) für verschieden Gasgruppen bei Normbedingungen (0°C, 1013.25 mbar), wobei das korrelierte Dichteverhältnisse mit einem Verfahren beziehungsweise mit einer Messvorrichtung gemäss vorliegender Erfindung ermittelt wurde. Als Referenzgas wurde ein typisches H-Erdgas verwendet.

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Messung des Druckabfalls oder der Druckzunahme in einem Gasreservoir, wobei das Gas durch eine kritische Düse fliesst, sowie Wärmeleitfähigkeits- und Durchflussmessung mit Hilfe eines mikrothermischen Sensors, und Datenvalidierung durch Aufsummieren der Durchflusswerte". Die verwendeten Komponenten sind kostengünstig, wodurch neue Märkte erschlossen werden können, in denen heute aus Kostengründen keine Gasqualitätssensoren eingesetzt werden. Von der Genauigkeit her sind nur wenig Einbussen gegenüber teureren, kommerziell erhältlichen Geräten zu erwarten, da hier ebenfalls mindestens drei voneinander unabhängige Messwerte für die Korrelation verwendet werden.

Die Erfindung umfasst in einer zweiten Ausführungsform die Verwendung eines Gasreservoirs und einer kritischen Düse zur Bestimmung physikalischer Eigenschaften und/oder brenntechnisch relevanter Grössen eines Gases oder Gasgemisches, bzw. ein Verfahren, in dem ein Gasreservoir und eine kritische Düse zur Bestimmung physikalischer Eigenschaften und/oder brenntechnisch relevanter Grössen eines Gases oder Gasgemisches verwendet werden, wobei das Gas oder Gasgemisch unter Druck aus dem Gasreservoir durch die kritische Düse fliesst, wobei der Druckabfall im Gasreservoir als Funktion der Zeit gemessen wird, aus den Messwerten des Druckabfalls ein von physikalischen Eigenschaften des Gases oder Gasgemisches abhängiger Gaseigenschaftsfaktor $\Gamma^*$ bestimmt wird, der zum Beispiel aus einer Zeitkonstante des Druckabfalls abgeleitet wird, und aus dem Gaseigenschaftsfaktor $\Gamma^*$ mittels Korrelation eine gesuchte physikalische Eigenschaft oder brenntechnisch relevante Grösse ermittelt wird.

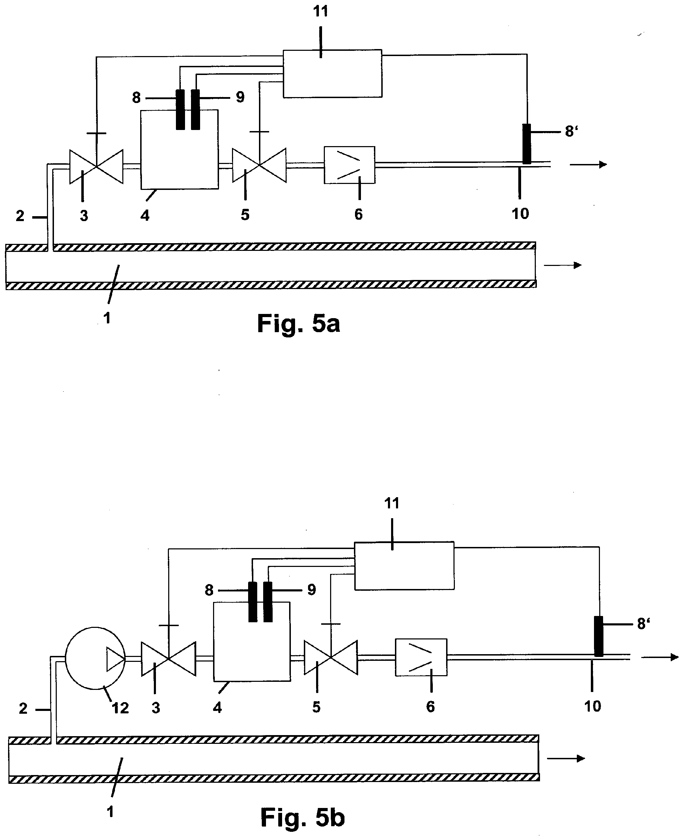

Fig. 5a zeigt ein Ausführungsbeispiel des schematischen Aufbaus einer Messvorrichtung gemäss der zweiten Ausführungsform der Erfindung im Fall, in dem die Gashauptleitung 1 einen Druck aufweist, der höher als der kritische Druck für die kritische Düse 6 der Messvorrichtung ist (Hochdruckvariante). Im Ausführungsbeispiel umfasst die Messvorrichtung zusätzlich zur kritischen Düse 6 eine Auswerteeinheit 11, die zur Ausführung eines Verfahrens gemäss der zweiten Ausführungsform der Erfindung eingerichtet ist, und ein Gasreservoir 4, das mit einem Drucksensor 8 versehen ist, wobei das Gasreservoir 4 für die Messung mit der kritischen Düse 6 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel des Verfahrens zur Bestimmung physikalischer Eigenschaften und/oder brenntechnisch relevanter Grössen eines Gases oder Gasgemisches gemäss der zweiten Ausführungsform der Erfindung wird im Folgenden anhand von Fig. 5a beschrieben. In diesem Ausführungsbeispiel fliesst das Gas oder Gasgemisch aus dem Gasreservoir 4 durch die kritische Düse 6. Der Druckabfall im Gasreservoir 4 wird als Funktion der Zeit gemessen und aus den Messwerten des Druckabfalls ein von einer Gruppe von physikalischen Eigenschaften des Gases oder Gasgemisches abhängiger Gaseigenschaftsfaktor F* bestimmt, wobei der Gaseigenschaftsfaktor zum Beispiel aus einer Zeitkonstante des Druckabfalls abgeleitet wird. Weiter wird aus dem Gaseigenschaftsfaktor $\Gamma^*$ mittels Korrelation eine gesuchte physikalische Eigenschaft oder brenntechnisch relevante Grösse ermittelt.

Vorteilhafterweise werden mit der zweiten Ausführungsform der Erfindung binäre Gasgemische auf ihren Anteil der beiden das Gasgemisch bildenden Komponenten analysiert, da der Gaseigenschaftsfaktor $\Gamma^*$ intrinsisch eine stetige Funktion der Gasanteile x% bzw. (1-x%) ist. Mit Kenntnis des Anteil x% bzw. (1-x%) können anschliessend aus Tabellenwerken oder mittels entsprechenden Berechnungsprogrammen physikalische Eigenschaften und/oder brenntechnisch relevanter Grösse des binären Gasgemisches bestimmt werden. Natürlich ist auch die direkte Korrelation dieser physikalische Eigenschaften und/oder brenntechnisch relevanten Grösse des binären Gasgemisches mit dem Gaseigenschaftsfaktor $\Gamma^*$ möglich.

In einer Ausführungsvariante des Verfahrens wird somit der prozentuale Anteil der einen Komponente in einem binären Gasgemisch bestimmt, wobei die zu korrelierende Grösse entweder dem Zusammensetzungsanteil der einen Komponente (x%) und/oder einer beliebig anderen physikalische Eigenschaft des binären Gasgemisches entspricht.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet. Das Einlassventil und Auslassventil können über eine Steuereinheit geöffnet werden. Fallweise kann auch die Auswerteinheit 11, wie in Fig. 5a gezeigt, die Steuerung des Einlassventils und Auslassventils übernehmen. Dann wird das Auslassventil 5 geschlossen und das Gasreservoir 4, dessen Volumeninhalt V bekannt ist, füllt sich, bis das Einlassventil 3 geschlossen wird. Druck p und Temperatur T im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} = \frac{p}{1013.25\,mbar} \cdot \frac{273.15\,K}{T} \cdot V \quad . \qquad (17)$$

Ist der Druck p im Gasreservoir 4 grösser als der Druck $p_{krit}$, der benötigt wird, damit die Düse 6 kritisch betrieben werden kann, kann das Auslassventil 5 wieder geöffnet werden. Vorzugsweise ist der Druck p im Gasreservoir um mehrere bar höher als $p_{krit}$, sodass die Druckabfallmessung über diesen Bereich der Drucküberhöhung vollzogen werden kann, ohne dass die Düse 6 nicht mehr kritisch betrieben ist. Jetzt wird das Auslassventil 5 wieder geschlossen, womit die Druckabfallmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Druckabfallmessung ist der zeitabhängige Druck p(t) und die zeitabhängige Temperatur T(t) im Druckreservoir 4 gemessen und von der Auswerteeinheit 11 aufgezeichnet worden. Mit diesen Daten werden in der Auswerteeinheit die Zeitkonstante $\tau$ in Gleichung (6) bzw. die Proportionalitätskonstante in Gleichung (6') und der Gaseigenschaftsfaktor $\Gamma^*$ in Gleichung (7) bzw. (7')bestimmt.

In der Auswerteeinheit 11 erfolgt nun, je nach gewünschter, brenntechnisch relevanter Grösse Q, deren Berechnung anhand der Gleichung (15) mit zuvor ermittelter Korrelationsfunktion $Q_{corr} = f_{corr}(\Gamma^*)$.

Bei Bedarf kann zudem, wie in Fig. 5b gezeigt, ein Verdichter 12' vorgesehen sein, der beispielsweise einlassseitig des Gasreservoirs 4 angeordnet ist, um den Druck im Gasreservoir zu erhöhen.

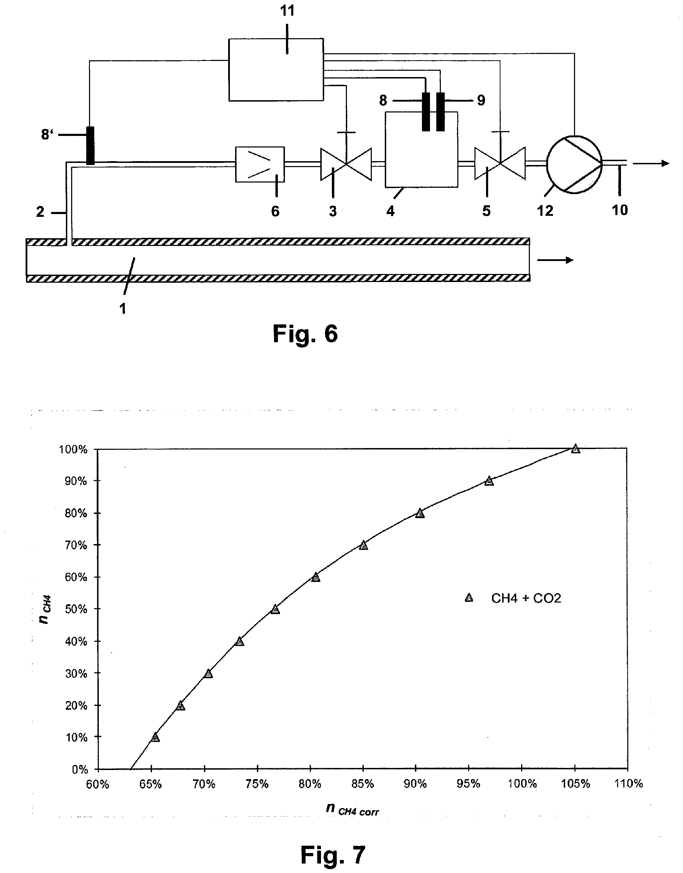

Fig. 6 zeigt ein zweites Ausführungsbeispiel des schematischen Aufbaus einer Messvorrichtung gemäss der zweiten Ausführungsform der Erfindung, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss der zweiten Ausführungsform der Erfindung eingerichtet ist, und eine kritische Düse 6, wobei das Gasreservoir 4 für die Messung mit der kritischen Düse 6 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein weiteres Ausführungsbeispiel des Verfahrens zur Bestimmung physikalischer Eigenschaften und/oder brenntechnisch relevanter Grössen eines Gases und Gasgemisches gemäss der zweiten Ausführungsform der Erfindung wird im Folgenden anhand von Fig. 6 beschrieben. In diesem Ausführungsbeispiel fliesst das Gas oder Gasgemisch unter Druck durch die kritische Düse 6 in das Gasreservoir 4. Die Druckzunahme im Gasreservoir 4 wird als Funktion der Zeit gemessen und aus den Messwerten der Druckzunahme ein von einer Gruppe von physikalischen Eigenschaften des Gases oder Gasgemisches abhängiger Gaseigenschaftsfaktor $\Gamma^*$ bestimmt, wobei der Gaseigenschaftsfaktor zum Beispiel aus einer Proportionalitätskonstante der Druckzunahme abgeleitet wird. Aus dem Gaseigenschaftsfaktor $\Gamma^*$ wird mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise wird der Druck im Gasreservoir 4 vorgängig soweit erniedrigt, zum Beispiel mit einer Vakuumpumpe 12, dass die kritische Düse 6 kritisch betrieben werden kann, d.h. der Druck im Gasreservoir weniger als die Hälfte des Drucks vor der kritischen Düse beträgt. Es ist kein Hochvakuum erforderlich: Solang der Druck $p$ und die Temperatur $T$ im Gasreservoir 4 gemessen werden, kann immer berechnet werden, welches Normvolumen an Gas in das Gasreservoir geflossen ist. Es ist jedoch von Vorteil, wenn der Druck um einen Faktor niedriger ist als es für kritische Verhältnisse nötig wäre, da dann entsprechend länger gemessen werden kann, was eine genauere Bestimmung der Proportionalitätskonstante ermöglicht.

Für weitere Einzelheiten zum Verfahren, die bei Bedarf verwendet werden können, wird auf die Beschreibung des ersten Ausführungsbeispiels verwiesen, wobei gegebenenfalls der Begriff "Druckabfall" durch "Druckzunahme" ersetzt werden muss.

Fig. 7 zeigt eine Darstellung des direkt gemessenen Methananteils $n_{CH4}$ (y-Achse) in Abhängigkeit des korrelierten Methananteils $n_{CH4\,corr}$ (x-Achse) für ein aus Methan und Kohlendioxid bestehendes, binäres Roh-Biogas bei Normbedingungen (0°C, 1013.25 mbar), wobei der korrelierte Methananteil mit einem Verfahren beziehungsweise mit einer Messvorrichtung gemäss der zweiten Ausführungsform der Erfindung ermittelt wurde. Als Referenzgas wurde ein typisches H-Erdgas verwendet. Die gesuchte Grösse Q (hier der Methananteil $n_{CH4\,corr}$ in x%) wird vorteilhafterweise mittels der Korrelationsfunktion $Q_{corr} = a + b \cdot \Gamma^* + c \cdot \Gamma^{*2} + d \cdot \Gamma^{*3}$ ermittelt, wobei in dem in der Darstellung gezeigten Bespiel numerisch a = -7.82, b = 22.7, c = -20.4 und d = 6.45.

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Messung des Druckabfalls oder der Druckzunahme in einem Gasreservoir, wobei das Gas durch eine kritische Düse fliesst. Die verwendeten Komponenten sind kostengünstig, wodurch neue Märkte erschlossen werden können, in denen heute aus Kostengründen keine Gasqualitätssensoren eingesetzt werden. Von der Genauigkeit her sind gewisse Einbussen gegenüber teureren, kommerziell erhältlichen Geräten zu erwarten, da hier anstatt drei nur ein unabhängiger Messwert für die Korrelation verwendet wird.

Zusätzlich umfasst die Erfindung in einer dritten Ausführungsform die Verwendung eines Gasreservoirs und eines für ein bestimmtes Kalibriergas oder -Gasgemisch kalibrierten mikrothermischen Sensors zur Bestimmung physikalischer Eigenschaften und/oder brenntechnisch relevanter Grössen eines Gases oder Gasgemisches, bzw. ein Verfahren, in dem ein Gasreservoir und ein für ein bestimmtes Kalibriergas oder -Gasgemisch kalibrierter mikrothermischer Sensor zur Bestimmung physikalischer Eigenschaften und/oder brenntechnisch relevanter Grössen eines Gases oder Gasgemisches verwendet werden, wobei das Gas oder Gasgemisch unter Druck aus dem Gasreservoir über den mikrothermischen Sensor fliesst, wobei der mit dem für ein bestimmtes Kalibriergas oder -Gasgemisch kalibrierte mikrothermische Sensor bestimmte Volumenfluss $v_x \cdot A$ aufsummiert und mit dem aus dem Gasreservoir ausgeflossenen Gasvolumen verglichen wird, aus dem Vergleich der beiden Volumina ein von physikalischen Eigenschaften des Gases oder Gasgemisches abhängiger Gaseigenschaftsfaktor $S/v'_x$ bestimmt wird, in dem $v'_x$ die aus dem ausgeflossenen Gasvolumen bestimmte Fliessgeschwindigkeit bezeichnet, und aus dem Gaseigenschaftsfaktor, der zum Beispiel durch

$$S \, / \, v'_x = c_p \cdot \rho / \lambda$$

gegeben sein kann (siehe Gleichung (9)), mittels Korrelation eine gesuchte physikalische Eigenschaft oder brenntechnisch relevante Grösse ermittelt wird.

Die oben beschriebene dritte Ausführungsform der Erfindung kann auch als eigenständige Erfindung betrachtet werden.

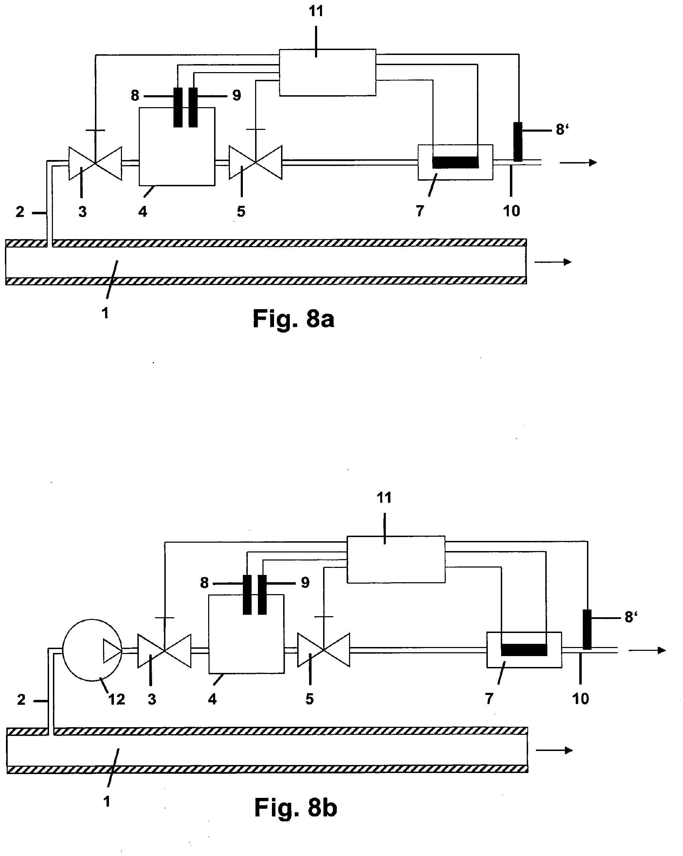

Fig. 8a zeigt ein Ausführungsbeispiel des schematischen Aufbaus einer Messvorrichtung gemäss der dritten Ausführungsform, welche nicht Teil der Erfindung ist, im Fall, in dem die Gashauptleitung 1 unter Druck steht (Hochdruckvariante). Im Ausführungsbeispiel umfasst die Messvorrichtung eine Auswerteeinheit 11, die zur Ausführung eines Verfahrens gemäss der dritten Ausführungsform der Erfindung eingerichtet ist, ein Gasreservoir 4, das mit einem Drucksensor 8 versehen ist, und einen mikrothermischen Sensor 7 zur Messung des Durchflusses und der Wärmeleitfähigkeit, wobei das Gasreservoir 4 für die Messung mit dem mikrothermischen Sensor 7 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel des Verfahrens zur Bestimmung physikalischer Eigenschaften und/oder brenntechnisch relevanter Grössen eines Gases und Gasgemisches gemäss der dritten Ausführungsform, welche nicht Teil der Erfindung ist, wird im Folgenden anhand von Fig. 8a beschrieben. In dem Verfahren fliesst das Gas oder Gasgemisch unter Druck aus dem Gasreservoir 4 über den für ein bestimmtes Kalibriergas oder -Gasgemisch kalibrierten mikrothermischen Sensor 7, wobei der Volumenfluss $v_x \cdot A$ aufsummiert und mit dem aus dem Gasreservoir ausgeflossenen Gasvolumen verglichen wird, aus dem Vergleich der beiden Volumina ein von physikalischen Eigenschaften des Gases oder Gasgemisches abhängiger Gaseigenschaftsfaktor $S \, / \, v'_x$ bestimmt wird, in dem $v'_x$ die aus dem ausgeflossenen Gasvolumen bestimmte Fliessgeschwindigkeit bezeichnet, und aus dem Gaseigenschaftsfaktor, der zum Beispiel durch

$$S \, / \, v'_x = c_p \cdot \rho / \lambda$$

gegeben sein kann (siehe Gleichung (9)), mittels Korrelation eine gesuchte physikalische Eigenschaft oder brenntechnisch relevante Grösse ermittelt wird.

In einer vorteilhaften Ausführungsform des Verfahrens wird mit Hilfe des mikrothermischen Sensors 7 zusätzlich die Wärmeleitfähigkeit $\lambda$ des Gases oder Gasgemisches bestimmt.

Vorteilhafterweise werden mit der dritten Ausführungsform, welche nicht Teil der Erfindung ist, Erdgasgemische auf ihre Zugehörigkeit zu den H-Gasen bzw. L-Gasen untersucht (Gase mit hohem (High) bzw. niedrigem (Low) Brennwert), da der Gaseigenschaftsfaktor, der zum Beispiel durch $S \, / \, v'_x = c_p \cdot \rho / \lambda$ gegeben sein kann (siehe Gleichung (9)), dem Kehrwert der Wärmediffusivität des Gasgemisches entspricht, anhand derer zusammen mit der Wärmeleitfähigkeit $\lambda$, die mit dem mikrothermischen Sensor separat gemessen werden kann, eine Unterscheidung der H- bzw. L-Gasgruppe möglich wird.

Die Klassenzugehörigkeit eines Erdgasgemisches zur H- bzw. L-Gas Gruppe kann beispielsweise bestimmt werden, indem der Gaseigenschaftsfaktor ($S \, / \, v'_x$) mit dem Kehrwert der Wärmediffusivität $c_p \cdot \rho / \lambda$ identifiziert wird, und indem bei gegebener Wärmeleitfähigkeit die Zuteilung anhand eines Grenzwerts für die Wärmediffusivität erfolgt, oberhalb dessen ein Gasgemisch als L-Gas und unterhalb dessen als H-Gas klassifiziert wird.

In einer Ausführungsvariante des Verfahrens wird somit mit Hilfe des mikrothermischen Sensors 7 zusätzlich die Wärmeleitfähigkeit $\lambda$ des Gases oder Gasgemisches bestimmt und zusammen mit dem Gaseigenschaftsfaktors $S \, / \, v'_x = c_p \cdot \rho / \lambda$ eine Einteilung des gemessenen Gases in H- beziehungsweise L-Gas vorgenommen.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet. Das Einlassventil und Auslassventil können über eine Steuereinheit geöffnet werden. Fallweise kann auch die Auswerteeinheit 11, wie in Fig. 8a gezeigt, die Steuerung des Einlassventils und Auslassventils übernehmen. Dann wird das Auslassventil 5 geschlossen und das Gasreservoir 4, dessen Volumeninhalt V bekannt ist, füllt sich, bis das Einlassventil 3 geschlossen wird. Druck $p$ und Temperatur $T$ im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} = \frac{p}{1013.25\,mbar} \cdot \frac{273.15\,K}{T} \cdot V \;. \qquad (17)$$

Nun kann das Auslassventil 5 wieder geöffnet werden. Vorzugsweise ist der Druck $p$ im Gasreservoir 4 um soviel höher als der Druck nach dem Gasreservoir, dass die Zeitspanne, in der das Gas aus dem Gasreservoir 4 über den mikrothermischen Sensor 7 fliesst, lange genug ist, um den Volumenfluss $v_x \cdot A$ genügend genau aufsummieren zu können. Jetzt wird das Auslassventil 5 wieder geschlossen, womit die Durchflussmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Durchflussmessung sind mit dem mikrothermischen Sensor 7 Durchflussdaten gemessen und von der Auswerteeinheit 11 aufgezeichnet worden, um den Faktor S in Gleichung (9) zu bestimmen. Da Ein- und Auslassventil nach der Durchflussmessung geschlossen sind, fliesst kein Gas mehr über den mikrothermischen Sensor 7. Jetzt kann die Messung der Wärmeleitfähigkeitsmessung $\lambda$ erfolgen. Wiederum von der Auswerteeinheit aufgezeichnet, wird die Wärmeleitfähigkeit $\lambda$ mit Hilfe der Lösung von Gleichung (12) bestimmt.

Mit diesen Daten wird in der Auswerteeinheit 11 der Volumenfluss zum Volumen $V_{sum}$ aufsummiert und mit dem aus dem Gasreservoir ausgeflossenen Gasvolumen $V_{diff}$ verglichen. Aus dem Vergleich der beiden Volumina kann nun ein von physikalischen Eigenschaften des Gases oder Gasgemisches abhängiger Gaseigenschaftsfaktor $S/v'_x$ bestimmt wird, in dem $v'_x$ die aus dem ausgeflossenen Gasvolumen bestimmte Fliessgeschwindigkeit bezeichnet. Zweckmässigerweise werden die Volumina für den Vergleich mittels Gleichung (17) auf Normbedingungen umgerechnet, so dass $v'_x$ durch

$$v'_x = v_x \cdot V_{diff}^{norm} / V_{sum}^{norm} \qquad (18)$$

gegeben ist mit dem auf Normbedingungen umgerechneten, ausgeflossenen Gasvolumen $V_{diff}^{norm}$ und dem auf Normbedingungen umgerechneten, aufsummierten Volumen $V_{sum}^{norm}$. Danach, je nach gewünschter, brenntechnisch relevanter Grösse Q, erfolgt nun in der Auswerteeinheit 11 deren Berechnung anhand der Gleichung (15) mit zuvor ermittelter Korrelationsfunktion $Q_{corr} = f_{corr}(S/v'_x)$ oder der Wert von $S/v'_x$ wird dazu benutzt, um zusammen mit der Wärmeleitfähigkeit $\lambda$ ein Erdgasgemisch der Kategorie H-bzw. L-Gas zuzuordnen.

Bei Bedarf kann zudem, wie in Fig. 8b gezeigt, ein Verdichter 12' vorgesehen sein, der beispielsweise einlassseitig des Gasreservoirs 4 angeordnet ist, um den Druck im Gasreservoir zu erhöhen.

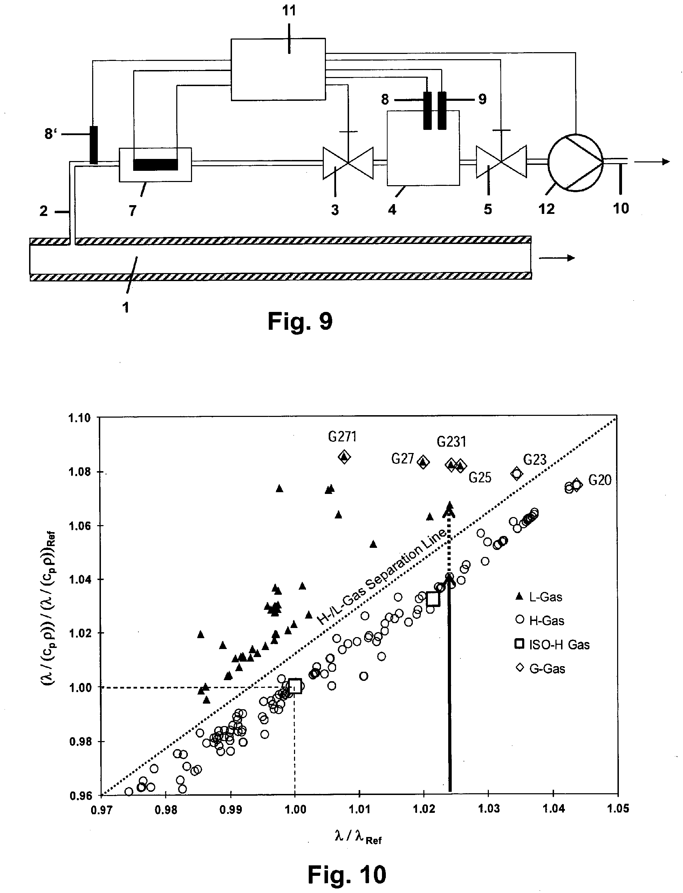

Fig. 9 zeigt ein zweites Ausführungsbeispiel des schematischen Aufbaus einer Messvorrichtung gemäss der dritten Ausführungsform, welche nicht Teil der Erfindung ist, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss der dritten Ausführungsform, welche nicht Teil der Erfindung ist, eingerichtet ist und einen mikrothermischen Sensor 7 zur Messung des Durchflusses und der Wärmeleitfähigkeit, wobei das Gasreservoir 4 für die Messung mit dem mikrothermischen Sensor 7 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein weiteres Ausführungsbeispiel des Verfahrens zur Bestimmung physikalischer Eigenschaften und/oder brenntechnisch relevanter Grössen eines Gases und Gasgemisches gemäss der dritten, welche nicht Teil der Erfindung ist, wird im Folgenden anhand von Fig. 9 beschrieben. In diesem Ausführungsbeispiel fliesst das Gas oder Gasgemisch unter einem Druck, der typisch soviel höher als der Druck nach dem Gasreservoir ist, dass die Zeitspanne, in der das Gas aus dem Gasreservoir 4 über den mikrothermischen Sensor 7 fliesst, lange genug ist, um den Volumenfluss $v_x \cdot A$ genügend genau aufsummieren zu können. Der aufsummierte Volumenfluss $V_{sum}$ wird mit dem aus dem Gasreservoir ausgeflossenen Gasvolumen $V_{diff}$ verglichen, aus dem Vergleich der beiden Volumina ein von physikalischen Eigenschaften des Gases oder Gasgemisches abhängiger Gaseigenschaftsfaktor $S / v'_x$ bestimmt, in dem $v'_x$ die aus dem ausgeflossenen Gasvolumen bestimmte Fliessgeschwindigkeit bezeichnet, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt.

In einer vorteilhaften Ausführungsform des Verfahrens wird mit Hilfe des mikrothermischen Sensors 7 die Wärmeleitfähigkeit $\lambda$ des Gases oder Gasgemisches bestimmt und beispielsweise zusammen mit dem Gaseigenschaftsfaktors $S / v'_x = c_p \cdot \rho / \lambda$ eine Einteilung des gemessenen Gases in H- beziehungsweise L-Gas vorgenommen.

Für weitere vorteilhafte Ausführungsformen und -varianten des Verfahrens und für Einzelheiten zum Verfahren, die bei Bedarf verwendet werden können, wird auf die vorangehenden Abschnitte der Beschreibung verwiesen, wobei gegebenenfalls der Begriff "Druckabfall" durch "Druckzunahme" ersetzt werden muss.

Fig. 10 zeigt eine Darstellung, wie anhand bekannter Wärmeleitfähigkeiten $\lambda$ (x-Achse) und Wärmediffusivitäten $\lambda/(c_p\rho)$, auch Temperaturleitfähigkeiten genannt, (y-Achse) eine Unterscheidung in H- bzw. L-Gas getroffen werden kann. L-Gase oberhalb der H-/L-Gas Trennlinie (H-/L-Gas Separation Line) haben typischerweise höhere Wärmediffusivitäten als H-Gase bei gleicher Wärmeleitfähigkeit unterhalb der Trennlinie (doppelter Pfeil bei x $\approx$1.024). Da der Gaseigenschaftsfaktor $S / v'_x = c_p \cdot \rho / \lambda$ im Wesentlichen der Kehrwert der Wärmediffusivität des Gasgemisches ist, kann demnach mit zusätzlich gemessener Wärmeleitfähigkeit $\lambda$ die Unterscheidung H- bzw. L-Gas getroffen werden. Alle Werte sind bei Normbedingungen (0°C, 1013.25 mbar) gezeigt. Als Referenzgas wurde ein typisches H-Erdgas verwendet (gestrichelte Linie bei der Koordinate (1.00,1.00).

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Wärmeleitfähigkeits- und Durchflussmessung mit Hilfe eines mikrothermischen Sensors, Aufsummieren der Durchflusswerte und Vergleich mit einem Volumenausfluss aus einem Referenzvolumen. Zuteilung der Erdgase zur H-Gas bzw. L-Gas Gruppe ". Die verwendeten Komponenten sind kostengünstig, wodurch neue Märkte erschlossen werden können, in denen heute aus Kostengründen keine Gasqualitätssensoren eingesetzt werden. Von der Genauigkeit her sind nur wenig Einbussen gegenüber teureren, kommerziell erhältlichen Geräten zu erwarten, da hier anstatt drei noch zwei voneinander unabhängige Messwerte für die Korrelation verwendet werden.

**Patentansprüche**

1. Verwendung eines Gasreservoirs (4) und einer kritischen Düse (6) zur Bestimmung physikalischer Eigenschaften und/oder brenntechnisch relevanter Grössen eines Gases oder Gasgemisches, wobei:

   - das Gas oder Gasgemisch unter Druck aus dem Gasreservoir (4) durch die kritische Düse (6) fliesst;
   - der Druckabfall im Gasreservoir (4) als Funktion der Zeit gemessen wird;
   - aus den Messwerten des Druckabfalls ein von physikalischen Eigenschaften des Gases oder Gasgemisches abhängiger Gaseigenschaftsfaktor ($\Gamma$*) bestimmt wird, der aus einer Zeitkonstanten des Druckabfalls abgeleitet wird, wobei von einem exponentiellen Abfall des gemessenen Druckes ausgegangen wird; und
   - aus dem Gaseigenschaftsfaktor ($\Gamma$*) mittels Korrelation eine gesuchte physikalische Eigenschaft oder brenntechnisch relevante Grösse ermittelt wird.

2. Verfahren nach Anspruch 1, in welchem der prozentuale Anteil der einen Komponente in einem binären Gasgemisch bestimmt wird, wobei die zu korrelierende Grösse entweder dem Zusammensetzungsanteil der einen Komponente (x%) und/oder einer beliebig anderen physikalischen Eigenschaft des binären Gasgemisches entspricht.

3. Verfahren nach einem der vorangehenden Ansprüche, in welchem binäre Gasgemische auf ihren Anteil der beiden das Gasgemisch bildenden Komponenten analysiert werden, wobei der Gaseigenschaftsfaktor ($\Gamma^*$) intrinsisch eine stetige Funktion der Gasanteile (x% bzw. 1-x%) ist, und mit Kenntnis des Gasanteils (x% bzw. 1-x%) anschliessend aus Tabellenwerken oder mittels entsprechenden Berechnungsprogrammen physikalische Eigenschaften und/oder brenntechnisch relevante Grössen des binären Gasgemisches bestimmt werden.

4. Verfahren nach einem der vorangehenden Ansprüche, wobei die gesuchte physikalische Eigenschaft die Dichte oder die Wärmeleitfähigkeit oder die Wärmekapazität oder die Viskosität des Gases oder Gasgemisches ist, und/oder wobei die brenntechnisch relevante Grösse der Energieinhalt oder der Brennwert oder der Wobbe-Index oder die Methanzahl oder der Luftbedarf des Gases oder Gasgemisches ist.

5. Verfahren nach einem der vorangehenden Ansprüche, wobei die gesuchte physikalische Eigenschaft oder brenntechnisch relevante Grösse (Q) mittels einer Korrelationsfunktion $Q_{corr} = a+b\cdot \Gamma^* + c \cdot\Gamma^{*2} + d\cdot \Gamma^{*3}$ ermittelt wird, wobei *a, b,* c und *d* Konstanten sind.

6. Messvorrichtung zur Bestimmung physikalischer Eigenschaften und/oder brenntechnisch relevanter Grössen eines Gases oder Gasgemisches mit einer Auswerteeinheit (11), die zur Ausführung eines Verfahrens gemäss einem der Ansprüche 1 bis 5 eingerichtet ist, und mit einer kritischen Düse (6) und einem Gasreservoir (4), das mit einem Drucksensor (8) versehen ist, wobei das Gasreservoir für die Messung mit der kritischen Düse verbunden ist.

7. Messvorrichtung nach Anspruch 6 umfassend zusätzlich einen Verdichter (12'), um den Druck im Gasreservoir zu erhöhen, oder eine Vakuumpumpe (12), die mit dem Gasreservoir (4) verbunden ist, um einen Unterdruck im Gasreservoir zu erzeugen.

**Claims**

1. Use of a gas reservoir (4) and a critical nozzle (6) for determining physical properties and/or combustion-relevant quantities of a gas or gas mixture, wherein:

   - the gas or gas mixture flows under pressure from the gas reservoir (4) through the critical nozzle (6);
   - the pressure drop in the gas reservoir (4) is measured as a function of time;
   - a gas property factor ($\Gamma^*$) dependent on physical properties of the gas or gas mixture is determined from the measured values of the pressure drop, which is derived from a time constant of the pressure drop, wherein an exponential drop of the measured pressure is assumed; and
   - a desired physical property or combustion-relevant quantity is determined from the gas property factor ($\Gamma^*$) by means of correlation.

2. A method according to claim 1, in which the percentage of the one component is determined in a binary gas mixture, wherein the variable to be correlated corresponds either to the composition percentage of the one component (x%) and/or to any other physical property of the binary gas mixture.

3. The method according to any one of the preceding claims, in which binary gas mixtures are analyzed for their content of the two components forming the gas mixture, wherein the gas property factor ($\Gamma^*$) is intrinsically a continuous function of the gas content (x% or 1-x%), and with knowledge of the gas content (x% or 1-x%), physical properties and/or combustion-relevant quantities of the binary gas mixture are subsequently determined from sets of tables or by means of corresponding calculation programs.

4. The method according to any one of the preceding claims, wherein the desired physical property is the density or the thermal conductivity or the heat capacity or the viscosity of the gas or gas mixture, and/or wherein the combustion-relevant quantity is the energy content or the calorific value or the Wobbe index or the methane number or the air requirement of the gas or gas mixture.

5. The method according to any one of the preceding claims, wherein the desired physical property or combustion-relevant quantity (Q) is determined by means of a correlation function $Q_{corr} = a + b\cdot \Gamma^* + c \cdot - \Gamma^{*2} + d \cdot \Gamma^{*3}$, wherein *a, b, c,* and *d* are constants.

6. A measuring device for determining physical properties and/or combustion-relevant quantities of a gas or gas mixture, having an evaluation unit (11) which is equipped for carrying out a method according to any one of the claims 1 to 5, and having a critical nozzle (6) and a gas reservoir (4) which is provided with a pressure sensor (8), wherein the gas reservoir is connected to the critical nozzle for the measurement.

**7.** The measuring device according to claim 6 additionally comprising a compressor (12') to increase the pressure in the gas reservoir or a vacuum pump (12) which is connected to the gas reservoir (4) to generate a negative pressure in the gas reservoir.

**Revendications**

**1.** Utilisation d'un réservoir de gaz (4) et d'une buse critique (6) pour déterminer les propriétés physiques et/ou les grandeurs, pertinentes en termes de combustion, d'un gaz ou d'un mélange gazeux, dans laquelle :

- le gaz ou le mélange gazeux s'écoule sous pression à partir du réservoir de gaz (4) en passant par la buse critique (6) ;
- la chute de pression dans le réservoir de gaz (4) est mesurée en fonction du temps ;
- un facteur de propriété de gaz ($\Gamma^*$) dépendant des propriétés physiques du gaz ou du mélange gazeux est déterminé à partir des valeurs mesurées de la chute de pression, ledit facteur étant dérivé d'une constante de temps de la chute de pression, en supposant une chute exponentielle de la pression mesurée ; et
- une propriété physique recherchée ou une grandeur pertinente en termes de combustion est déterminée à partir du facteur de propriété de gaz ($\Gamma^*$) au moyen d'une corrélation.

**2.** Procédé selon la revendication 1, dans lequel le pourcentage d'un composant dans un mélange gazeux binaire est déterminé, dans lequel la grandeur à corréler correspond à la proportion dudit composant (x%) et/ou à une autre propriété physique quelconque du mélange gazeux binaire.

**3.** Procédé selon l'une quelconque des revendications précédentes, dans lequel des mélanges gazeux binaires sont analysés afin de déterminer la proportion des deux composants formant le mélange gazeux, dans lequel le facteur de propriété de gaz ($\Gamma^*$) est intrinsèquement une fonction continue des proportions de gaz (x% ou 1-x%), et, en connaissance de la proportion de gaz (x% ou 1-x%), des propriétés physiques et/ou des grandeurs, pertinentes en termes de combustion, du mélange gazeux binaire sont ensuite déterminées à partir de tables ou au moyen de programmes de calcul correspondants.

**4.** Procédé selon l'une quelconque des revendications précédentes, dans lequel la propriété physique recherchée est la densité ou la conductivité thermique ou la capacité thermique ou la viscosité du gaz ou du mélange gazeux, et/ou dans lequel la grandeur pertinente en termes de combustion est la capacité énergétique ou le pouvoir calorifique ou l'indice de Wobbe ou l'indice de méthane ou la demande d'air du gaz ou du mélange gazeux.

**5.** Procédé selon l'une quelconque des revendications précédentes, dans lequel la propriété physique recherchée ou la grandeur (Q) pertinente en termes de combustion est déterminée au moyen d'une fonction de corrélation $Q_{corr} = a + b \cdot \Gamma^* + c \cdot \Gamma^{*2} + d \cdot \Gamma^{*3}$, dans lequel $a$, $b$, $c$ et $d$ sont des constantes.

**6.** Dispositif de mesure permettant de déterminer des propriétés physiques et/ou des grandeurs, pertinentes en termes de combustion, d'un gaz ou d'un mélange gazeux, comprenant une unité d'évaluation (11) conçue pour mettre en œuvre un procédé selon l'une quelconque des revendications 1 à 5, et comprenant une buse critique (6) et un réservoir de gaz (4) muni d'un capteur de pression (8), dans lequel le réservoir de gaz est relié à la buse critique en vue de la mesure.

**7.** Dispositif de mesure selon la revendication 6, comprenant en outre un compresseur (12') afin d'augmenter la pression dans le réservoir de gaz, ou une pompe à vide (12) reliée au réservoir de gaz (4) afin de créer une dépression dans le réservoir de gaz.

Fig. 1a

Fig. 1b

**Fig. 2**

**Fig. 3**

**Fig. 4**

**Fig. 5a**

**Fig. 5b**

**Fig. 6**

**Fig. 7**

**Fig. 8a**

**Fig. 8b**

**Fig. 9**

**Fig. 10**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9902964 A1
- EP 0591639 A2
- EP 2015056 A1
- WO 2004036209 A1

**In der Beschreibung aufgeführte Nicht-Patentliteratur**

- **U. WERNEKINCK.** Gasmessung und Gasabrechung. Vulkan Verlag, 2009
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146
- **KERSON HUANG.** Statistical Mechanics. John Wiley & Sons, 1987